Europäisches Patentamt

European Patent Office

Office européen des brevets

(19)

(11) Veröffentlichungsnummer : **0 026 349**
**B1**

(12) **EUROPÄISCHE PATENTSCHRIFT**

(45) Veröffentlichungstag der Patentschrift :
16.02.83

(21) Anmeldenummer : 80105277.0

(22) Anmeldetag : 04.09.80

(51) Int. Cl.³ : **C 07 C 19/045, C 07 C 17/02**

(54) **Verfahren zur Herstellung von 1.2-Dichlorethan.**

(30) Priorität : 05.09.79 DE 2935884

(43) Veröffentlichungstag der Anmeldung :
08.04.81 Patentblatt 81/14

(45) Bekanntmachung des Hinweises auf die Patenterteilung : 16.02.83 Patentblatt 83/07

(84) Benannte Vertragsstaaten :
BE DE FR GB IT NL SE

(56) Entgegenhaltungen :
DE A 2 427 045
DE B 1 902 843
GB A 1 231 127
US A 2 929 852

(73) Patentinhaber : WACKER-CHEMIE GMBH
Prinzregentenstrasse 22
D-8000 München 22 (DE)

(72) Erfinder : Schmidhammer, Ludwig, Dr. Dipl.-Chem.
Pappelweg 5
D-8261 Haiming/Markt 1 (DE)
Erfinder : Strasser, Rudolf, Dr. Dipl.-Chem.
Lindacher Strasse 58
D-8263 Burghausen (DE)
Erfinder : Dummer, Gerhard, Dipl.-Chem.
Alzweg 9
D-8261 Burgkirchen/Alz (DE)

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von 1.2-Dichlorethan

Die Erfindung betrifft ein verbessertes Verfahren zur Herstellung von 1.2-Dichlorethan durch Chloraddition an Ethylen in flüssiger Phase. Insbesondere bezieht sich die Erfindung auf eine Kombination von chemischen und physikalisch-chemischen Maßnahmen zur Beherrschung der hochexothermen Reaktion und zur Vermeidung unerwünschter Nebenreaktionen.

Es ist bereits eine Anzahl von Verfahren bekannt geworden, die sich mit diesem Problemkreis beschäftigen.

Es wird meistenteils unter Normaldruck bei Temperaturen von 20 bis 70 °C gearbeitet. Die Reaktionswärme wird dabei direkt oder indirekt durch Kühlvorrichtungen abgeführt, die innerhalb oder außerhalb des Reactors installiert sind. Das gebildete Dichlorethan wird aus dem Reaktor flüssig abgezogen und zur Entfernung von unerwünschten Verunreinigungen, wie Eisenchlorid, Chlorwasserstoff, Chlor und höher chlorierten Kohlenwasserstoffen einer Wasser- und Laugenwäsche, einer aceotropischen Trocknung und einer Reindestillation unterworfen. Die dabei auftretenden Korrosions- und Abwässerprobleme und ebenso die Dichlorethanverluste sind bekannt.

Nach anderen Verfahren wird die Reaktion unter Siedebedingungen durchgeführt und das Produkt gasförmig abgezogen.

Die US-PS 2 929 852 und die DE-PS 1 112 504 beschreiben derartige Verfahren. Die Reaktionsenthalpie wird dabei zum Teil zur Fraktionierung des verdampften Dichlorethans verwendet und die Wärmebilanz dadurch ausgeglichen, daß der größte Teil des erhaltenen Produkts nach Kondensation wieder in den Reaktor zurück geleitet wird.

Bei Austritt großer Dampfmengen entweichen aber aus der Reaktionszone auch beträchtliche Mengen an nicht umgesetztem Chlor und Ethylen. Da die Löslichkeit von Chlor erheblich über der des Ethylens liegt, kommt es über den Kondensatrückstrom zu einem erhöhten Chlorangebot im Reaktor, während das Ethylen aus dem Prozeß ausgeschleust wird. Abgesehen davon, daß die Ethylenverluste das Verfahren unrationell machen, führen diese speziellen Reaktionsbedingungen unvermeidlich zu verstärkten Substitutionsreaktionen durch Chlor.

Da die Bildungswärme von Dichlorethan aus Chlor und Ethylen etwa 6,5 mal größer als die Dichlorethanverdampfungswärme ist, schlägt die GB-PS 1 231 127 vor, zusätzlich Dichlorethan aus anderen Quellen in das System einzuspeisen und zu rektifizieren, um damit überschüssige Reaktionswärme auszunutzen.

Gemäß DE-OS 1 618 273 wird zur Kontrolle der Reaktionsbedingungen ein internes Kühlungssystem im Reaktor installiert und das siedende Reaktionsgemisch zusätzlich über eine Umwälzpumpe im Kreislauf geführt.

Nach DE-OS 2 224 253 wird die Reaktion über einen externen Wärmeaustauscher kontrolliert, wobei die Zirkulation des Reaktionsmediums durch Ausnützen von Thermosiphon bzw. Gasauftriebseffekten aufrechterhalten wird. Ferner wird vorgeschlagen, kontinuierlich eine Teilmenge des zirkulierenden Reaktionsmediums auszuspülen und einer separaten Reinigung zuzuführen, um hochsiedende Verunreinigungen zu extrahieren. Dieser Verfahrensschritt ist aber unrationell, da dadurch die Anlagenkapazität, bezogen auf das Zielprodukt, bis zu 10 % reduziert wird.

Alle diese beschriebenen Verfahrensweisen, die ein siedendes Reaktionsmedium vorsehen, besitzen eine Reihe von Nachteilen. Insbesondere wirkt sich die Blasenbildung, die zum Teil durch die siedenden Bedingungen, zum Teil dadurch, daß die Reaktanten, Chlor und Ethylen, in gasförmigem Zustand eingeschleust werden, negativ aus : große Dampfblasen im Reaktionsmedium ergeben eine relativ kleine Gas-Flüssigkeits-Grenzfläche, an der die Hauptreaktion stattfindet. Es kommt so zu vermehrten Nebenreaktionen in der Gasphase. Bei ökonomisch wünschenswert hohen Durchsatzgeschwindigkeiten löst sich nicht alles Ethylen und neigt dazu, Gasblasen zu bilden, die, nicht umgesetzt, der Flüssigkeit entsteigen. Außerdem fördert eine Durchsatzsteigerung durch vermehrten Wärmeanfall ein erhöhtes Sieden, was sowohl zu einem Sicherheitsproblem, wie zur vermehrten Bildung von Nebenprodukten führen kann.

Die DE-OS 2 427 045 beschreibt daher ein Verfahren, in dem Ethylen und Chlor unter erhöhtem Druck in einem flüssigen, zirkulierenden Medium zur Reaktion gebracht werden. Das Reaktionsprodukt wird anschließend in eine Zone verminderten Drucks entspannt, wo es aufgrund der herrschenden Druck-Temperatur-Verhältnisse zum Teil verdampft und einer Rektifizierung zugeführt wird. Der verbleibende Rest wird in einem Kreislaufverfahren der Druckzone wieder zugeführt. Dadurch wird zwar erreicht, daß die Reaktion ohne Blasenbildung in einem nicht-siedenden Reaktionsmedium stattfindet, durch die zwangsläufig erhöhten Temperaturen im Reaktionsraum kommt es aber dennoch zu einer vermehrten Nebenproduktbildung : es entstehen vermehrt höher siedende, polychlorierte Kohlenwasserstoffe, da bei Temperaturerhöhung ganz allgemein die radikalisch verlaufende Substitutionschlorierung merklich zunimmt. Zudem begünstigt Druckerhöhung bekanntlich die Hydrochlorierungsreaktion. So kommt es, durch Anlagerung von Chlorwasserstoff, der bei den substituierenden Nebenreaktionen in Freiheit gesetzt wird, auch in verstärktem Maße zu niedrig-siedenden Verunreinigungen, wie Ethylchlorid. Die relativ hohen Temperaturen begünstigen weiter die dehydrochlorierende-Spaltung von 1.2-Dichlorethan. Sich intermediär bildendes Vinylchlorid wird durch Chlorwasserstoffaddition in den unerwünschten, destillativ relativ schwierig abzutrennenden Leichtsieder 1.1-Dichlorethan verwandelt. Dadurch muß ständig ein beträchtlicher Anteil an 1.2-Dichlorethan-haltigem Vorlauf aus dem System entfernt und einer separaten Dichlorethanrückgewinnung zugeführt werden. Zudem verlangen die erhöhten Druck- und Temperaturbedingungen, schon allein aus Sicherheitsgründen, einen erhöhten und kostspieligen Aufwand an Material- und Geräteausstattung.

Aufgabe der vorliegenden Erfindung war es nun, die oben geschilderten Nachteile zu vermeiden. Es wurde eine Kombination von Maßnahmen gefunden, die eine genau kontrollierte Reaktionsführung unter moderaten Reaktionsbedingungen ermöglicht, eine exakt ausgeglichene Bilanz von entstehender und abzuführender Wärme gewährleistet, und Nebenreaktionen weitgehend unterdrückt.

Gegenstand der Erfindung ist somit ein verbessertes Verfahren zur Herstellung von 1.2-Dichlorethan durch Chlorierung von Ethylen in der Flüssigphase in Gegenwart von Lewissäurekatalysatoren, wobei die Hauptmenge der freiwerdenden Reaktionswärme zur Verdampfung und Rektifizierung des Reaktionsprodukts ausgenützt wird, dadurch gekennzeichnet, daß die Reaktion bei Drücken von 0,3 bis 1,3 bar und Temperaturen von 50 bis 90 °C durchgeführt wird, mindestens die Hälfte des benötigten Chlors gelöst im gekühlten Reaktionsprodukt zum Einsatz kommt und das restliche Chlor gasförmig und/oder flüssig zugegeben wird, das Reaktionsprodukt vor der Chlorabsorption mit 0,000 1 bis 0,01 Gew.% o-Kresol, m-Kresol und/oder deren Mono- bzw. Dichlorderivaten einzeln und/oder im Gemisch versetzt wird und die Wärmebilanz ausgeglichen wird durch die Maßnahmen :

a) Verdampfen des Reaktionsprodukts

b) Rektifizieren des Reaktionsprodukts bei 3- bis 5-fachem Rücklauf, bezogen auf den Produktaustrag vom Kondensator in den Kolonnenkopf

c) Ausschleusen des Reaktionsprodukts

d) Kühlen des Reaktionsprodukts in Mengen vom 1,5- bis zum 10-fachen des Produktaustrags und

e) Beschicken des gekühlten Reaktionsprodukts mit Chlor und Rückführen des chlorhaltigen, gekühlten Reaktionsprodukts in den Reaktionsraum.

Die Reaktion wird vorzugsweise in Gegenwart eines geringen Überschusses an Chlor, bezogen auf die Menge umzusetzenden Ethylens, ausgeführt.

In einer besonders vorteilhaften Ausführungsform des Verfahrens wird im Reaktor ein Umwälzkreislauf aufrechterhalten und, als weitere Maßnahme, ein Teilstrom des gekühlten, mit o-Kresol, m-Kresol und/oder deren Mono- bzw. Dichlorderivaten versetzten Reaktionsprodukts zur Abgaswäsche verwendet und anschließend in die Reaktionszone zurückgeführt.

Das Reaktionssystem besteht aus einem Reaktorunterteil und einer seitlich aufgesetzten Rektifiziersäule. Die Rektifiziersäule kann aber auch auf dem Reaktorunterteil aufgesetzt sein. Der Rücklaufkondensator kann seitlich von der Rektifizierkolonne angebracht oder als Dephlegmator auf der Kolonne installiert sein.

Der Kopfdruck des Systems wird durch eine Stickstoffatmung konstant bei etwa 300 mm WS Überdruck gehalten.

Aufgrund der hydrostatischen Gegebenheiten stellt sich im Sumpf der Reaktionszone ein leichter Überdruck von etwa 0,2 bis 0,3 bar ein. Das gesamte Reaktionssystem kann aber auch unter vermindertem Druck betrieben werden, wobei sich aber insgesamt keine Vorteile ergeben ; der Vorteil gegenüber Hochdruckverfahren, die Wärmebilanz aufgrund der Tatsache günstiger ausgleichen zu können, daß die Verdampfungsenthalpie von 1.2-Dichlorethan mit fallendem Druck steigt, ist auch bei der angegebenen Obergrenze (1,3 bar) ausreichend gewahrt.

Zum Ausgleich der Wärmebilanz wird vom Kondensator etwa die 5-fache Menge verdampften Reaktionsprodukts, bezogen auf die Produktbildung in die Rektifiziersäule zurückgeführt. Aus dem Rektifizierteil wird Produkt entnommen und zur Absorption gasförmigen Chlors in ein Kreislaufsystem eingespeist. Die Produktmenge wird dabei so bemessen, daß 50 bis 100 % des für den Ethylenumsatz und zur Nachwäsche des Reaktionsabgases benötigten Chlors, bei gegebenem Druck und gegebener Temperatur, absorbiert wird. Das mit Chlor beladene Umlaufprodukt und das Ablaufprodukt aus dem Abgaswäscher werden anschließend zusammen mit dem restlichen Chlor in die Reaktionszone gebracht und mit einer in etwa stöchiometrischen Menge an Ethylen umgesetzt. Zur besseren Dispersion der Reaktionspartner wird aus dem Oberteil der Reaktionszone Produkt ohne Kühlung mittels einer Umwälzpumpe durch geeignete Ethylen- bzw. Chlorgasdiffusoren bzw. -Verteiler im Kreis gefahren. In einer vorteilhaften Ausführungsform ist der Umwälzteil mit separaten Einspeisungsvorrichtungen für Ethylen bzw. Chlor ausgestattet.

Die Einspeisung von Chlor in gelöster Form stellt eine besonders bevorzugte Maßnahme dar. Es wurde überraschenderweise festgestellt, daß die Nebenproduktbildung selbst dann stark unterdrückt werden kann, wenn das Reaktionsmedium nahezu siedet, falls mindestens die Hälfte des äquimolar zu Ethylen benötigten gasförmigen Chlors in gelöster Form eingesetzt wird.

Um zu verhindern, daß während der Chlorabsorption oder auf dem Weg von der Absorptionsvorrichtung zum Reaktor eine Reaktion von Chlor mit 1.2-Dichlorethan unter Bildung unerwünschter Substitutionsprodukte stattfindet, wird das Umlaufprodukt vor der Chlorabsorption mit den, vorzugsweise in Dichlorethan gelösten, hydroxylgruppenhaltigen Aromaten o-, m-Kresol bzw. deren Mono- oder Dichlorderivaten in Mengen von 0,000 1 bis 0,01 Gew.%, bezogen auf das Umlaufprodukt, einzeln oder im Gemisch, versetzt. Es ist gemäß der DE-PS 1 902 843 bekannt, daß derartige hydroxylgruppenhaltige Aromaten die Additionsreaktion von Chlor fördern. Daß aber derartig geringe Mengen dieser Kresole gleichzeitig die Substitutionsreaktion von Chlor mit dem gesättigten Chloralkan nahezu vollständig inhibieren, ist äußerst überraschend. Mit dem Umlaufprodukt gelangen nun diese hydroxylgruppenhaltigen Aromaten, nach Durchlaufen der Chlorabsorptionskolonne, in den Reaktor, wo ihre katalytische Wirkung für die Additionsreaktion zusammen mit der katalytischen Aktivität der üblicherweise verwendeten Lewissäuren, die im allgemeinen in Mengen von 0,005 bis 0,5 Gew.%, bezogen auf das Reaktionsmedium, eingesetzt werden, voll zur Entfaltung kommt.

Erfahrungsgemäß induzieren bereits entstandene Nebenprodukte durch die Änderung von Reaktionsparametern, wie z. B. Reaktionstemperatur oder durch das Auftreten von Fremdreaktionspartnern, wie z. B. HCl usw., die Bildung weiterer Nebenprodukte. Das erfindungsgemäße Verfahren ist deshalb insbesondere darauf zugeschnitten, durch eine Kombination von chemischen und physikalisch-chemischen Maßnahmen die Nebenproduktbildung von vornherein zu vermeiden. Das vorgeschlagene Verfahren erzielt damit nicht nur produktionstechnische Vorteile, sondern wird auch den aktuellen Erfordernissen des Umweltschutzes gerecht : So reduziert beispielsweise der geringe Anfall an Leichtsiedern die an die Rektifizier- und Kondensationseinrichtungen zu stellenden Anforderungen. Gleichzeitig werden aber auch durch günstige Emissionswerte Umweltbelastungen vermieden. Ebenso entschärft der geringe Anteil an höherchlorierten bzw. kondensierten, teerartigen Nebenprodukten die durch die erforderliche Laugenwäsche auftretenden Abwässerprobleme — es werden aber dadurch auch Produktionseinschränkungen und Ausbeuteverluste eingeschränkt.

Durch das erfindungsgemäße Verfahren gelingt es, 1.2-Dichlorethan in großer Ausbeute und in hoher Reinheit zu erzeugen. So erfüllt das erhaltene Produkt beispielsweise die für die Vinylchlorid-Produktion zu fordernden Qualitätsmaßstäbe.

Die an die Ausgangssubstanzen zu stellenden Reinheitskriterien sind relativ unkritisch : so kann reines Gas-Chlor, Flüssigchlor, aber auch Elektrolyse-Chlor mit einem üblichen Verunreinigungsgrad von ca. 10 % zum Einsatz kommen. Das eingesetzte Ethylen kann etwa 20 % Beimengungen, wie Methan, Ethan, Kohlenmonoxid, Stickstoff und dergleichen enthalten.

Es sei darauf hingewiesen, daß sich das erfindungsgemäße Verfahren in der, dem Fachmann geläufigen Weise auch auf die Chlorierung anderer Olefine, wie Propylen, Butylen und dergleichen übertragen läßt ; jedoch wird dies hier nicht beansprucht. Ebenso kann auch 1.2-Dichlorethan aus anderen Quellen mit aufgearbeitet werden.

Das erfindungsgemäße Verfahren wird nun anhand von Beispielen näher erläutert :

Beispiel 1

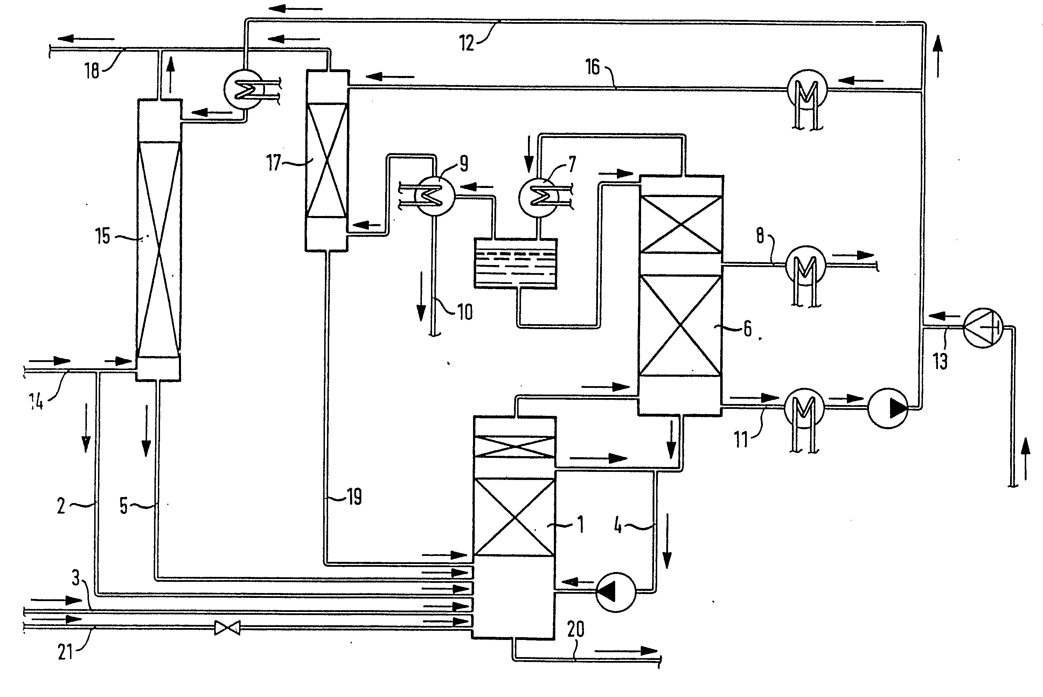

Der Verfahrensablauf wird in Figur 1 veranschaulicht.

Über Leitung 3 werden dem Reaktor 1 mengengeregelt 1 650 kg/Stunde Ethylen mit einer Temperatur von 40 °C und 3 bar Druck über einen internen Diffusor, der über Leitung 4 mit Umwälzprodukt aus dem Reaktoroberteil beaufschlagt ist, zugesetzt. Über Leitung 2 strömen mengengeregelt etwa 900 kg/Stunde Elektrolyse-Chlor mit einer Temperatur von 30 °C und 1,8 bar Druck über einen internen Gas-Chlorverteiler zu. Über Leitung 5 fließen 10,8 t/Stunde Umlaufprodukt zu, in dem bei Atmosphärendruck und − 10 °C 3 240 kg/Stunde Chlor gelöst enthalten sind. Der Druck im Reaktorunterteil beträgt 1,3 bar. Durch die Reaktion von Chlor mit Ethylen stellt sich im Reaktorunterteil eine Temperatur von etwa 95 °C unter Verdampfung des Reaktionsprodukts ein. Der Dampf steigt in die über dem Reaktor seitlich angeordnete, gepackte Rektifiziersäule 6 auf. Am Kopf der Fraktioniersäule befindet sich ein seitlich angeordneter Kondensator 7, der etwa 28 t/Stunde Rücklauf in die Kolonne liefert. Über Leitung 8 werden durch einen Seitenabzug kontinuierlich 5,6 t/Stunde hochreines 1.2-Dichlorethan mit einer Temperatur von 85 °C flüssig abgezogen und nach Kühlung in die Vinylchlorid-Anlage abgegeben. Über dem Seitenabzug ist demnach der Rücklauf 5-fach, darunter 4-fach, jeweils bezogen auf den Produktaustrag. Die Menge an im Kondensator 7 nicht kondensierbaren Anteilen beträgt 25 kg/Stunde und wird nach Kondensation im Wärmeaustauscher (9) und Abtrennung von Inertgasen über Leitung 10 abgezogen und einem anderen Verwendungszweck zugeführt. Dem Sumpf der Rektifizierkolonne 6 werden bei 86 °C über Leitung 11 kontinuierlich 13 t/Stunde Reaktionsprodukt entnommen, welches nach Kühlung mit Kühlwasser bzw. Sole über Leitung 13 mit 10 Gew. ppm o-Kresol, bezogen auf die Umlaufmenge, versetzt wird. Das Reaktionsprodukt wird nun über Leitung 12 einem Chlorabsorber 15 zugeführt, in den im Gegenstrom von unten über Leitung 14 3 600 kg/Stunde Elektrolyse-Chlor eingeleitet werden. Das mit Chlor beaufschlagte Reaktionsprodukt wird nun über Leitung 5 in den Reaktorunterteil eingebracht.

Ein kleiner Teilstrom des umlaufenden Reaktionsprodukts von etwa 2,2 t/Stunde wird nach Kühlung auf − 10 °C über Leitung 16 einem Abgaswäscher 17 aufgegeben, in dem das Reaktions- bzw. Rektifizierabgas nochmals gewaschen wird, bevor es mit dem Abgas aus der Chlorabsorption durch Leitung 18 über eine Stickstoffbeatmung ins Freie ausgeschleust wird. Die Stickstoffbeatmung hält einen konstanten Kopfdruck von 300 mm WS im System aufrecht. Die Temperatur im Ablauf des Kondensators 7 beträgt demnach 83 °C. Der Flüssigablauf des Wäschers 17 fließt mit einer Temperatur von 2 °C über Leitung 19 ebenfalls in den Reaktorunterteil. Über Leitung 20 werden diskontinuierlich etwa

alle 300 Stunden ca. 10 t Hochsieder enthaltendes Reaktionssumpfprodukt entnommen und einer separaten Aufarbeitung zugeführt (dieser Menge entsprechen ca. 0,6 Gew.% Sumpfprodukt, bezogen auf die Produktion).

Das aus dem Reaktionssystem abgeblasene Abgas enthält 1,6 kg/Stunde Ethylen und Chlor.

Der Ethylenumsatz beträgt demnach 99,9 % der Chlorumsatz 99,96 %.

Die um den Vorlauf korrigierten Ausbeuten betragen

für Ethylen   99,5 %
für Chlor       99,6 %.

Das Reinprodukt hat folgende Zusammensetzung :

> 99,99 Gew.% 1.2-Dichlorethan
< 10       Gew.ppm 1.1-Dichlorethan
< 10       Gew.ppm Ethylchlorid
< 10       Gew.ppm 1.1.2-Trichlorethan

Vorlaufzusammensetzung :

6 Gew.% Ethylchlorid
2 Gew.% 1.1-Dichlorethan
92 Gew.% 1.2-Dichlorethan

Sumpfzusammensetzung :

35,5 Gew.% 1.1.2-Trichlorethan
0,2 Gew.% chloriertes o-Kresol
8,0 Gew.% Tetra- und Pentachlorethane
56,3 Gew.% 1.2-Dichlorethan

Demnach liefert das erfindungsgemäße Verfahren folgende rechnerische Zusammensetzung an rohem Dichlorethan :

267 Gew.ppm Ethylchlorid
89 Gew.ppm 1.1-Dichlorethan
2 009 Gew.ppm 1.1.2-Trichlorethan
446 Gew.ppm Tetra- und Pentachlorethane
Rest 1.2-Dichlorethan

Beispiel 2

Es wird analog Beispiel 1 verfahren, wobei jedoch nur 2 250 kg/Stunde Chlor, gelöst im gekühlten Reaktionsprodukt, zum Einsatz kommen, während das restliche Chlor je zur Hälfte über Leitung 2 bzw. als Flüssig-Chlor über Leitung 21 in Mengen von je 1 125 kg/Stunde dem Reaktor 1 zugeführt werden. Der Kondensator 7 liefert 19,6 t/Stunde Rücklauf in die Kolonne 6. Entsprechend ist der Rücklauf über dem Seitenabzug 8 3,5-fach, darunter 2,5-fach, jeweils bezogen auf einen Produktabzug von 5,6 t/Stunde. Über Leitung 11 werden ca. 4,5 t/Stunde Reaktionsprodukt entnommen und bei Leitung 13 mit 5 Gew.ppm m-Kresol versetzt. Davon gelangen 2,2 t/Stunde, nach Tiefkühlung auf − 10 °C in den Wäscher 17, während das restliche Reaktionsprodukt nach Kühlung auf 0 °C dem Chlorabsorber zugeführt wird.

Sowohl die Produktzusammensetzung als auch die Ausbeuten entsprechen dem Zahlenmaterial aus Beispiel 1.

Beispiel 3

Es kommt die in Beispiel 1 beschriebene Apparatur zum Einsatz, wobei jedoch in der Abgasleitung vom Wärmetauscher 9 zum Abgaswäscher 17 ein Vakuumgebläse eingebaut ist, das im gesamten Reaktions- und Rektifiziersystem ein Teilvakuum von 0,35 bar aufrechterhält. Auf der Druckseite des Gebläses wird der im Wärmeaustauscher 9 nicht kondensierbare Abgasstrom in den Abgaswäscher 17 gefördert. Die Ausschleusung inerter Bestandteile erfolgt über Leitung 18.

1 400 kg/Stunde Ethylen, 750 kg/Stunde Gas-Chlor und 2 880 kg/Stunde in 12 500 kg/Stunde Reaktionsprodukt bei − 10 °C gelöstes Chlor, werden im Reaktor 1 umgesetzt. Der Druck im Reaktorunterteil beträgt etwa 0,45 bar. Die Gesamtumwälzmenge, in der (durch kontinuierliche Zudosierung über 13) 10 Gew.ppm o-Kresol gelöst enthalten sind, beträgt 14 500 kg/Stunde. Davon werden etwa 2 000 kg/Stunde für die Abgaswäsche benötigt, während der Rest nach Solekühlung dem Chlorabsorber aufgegeben wird. Der Ablaufstrom des Abgaswäschers 17 fließt mit einer Temperatur von 2 °C dem Reaktorunterteil zu. Durch die Reaktion von Chlor mit Ethylen in Gegenwart von etwa 2 000 ppm gelöstem $FeCl_3$ stellt sich eine Temperatur von etwa 60 °C ein. Das unter den gegebenen Druck- und Temperaturbedingungen verdampfende Reaktionsprodukt steigt in die über dem Reaktor seitlich angeordnete Rektifiziersäule 6 auf. Der Kondensator 7 am Kopf der Fraktioniersäule liefert einen Rücklauf von etwa 24,7 t/Stunde. Über Leitung 8 werden durch einen Seitenabzug kontinuierlich 4,94 t/h hochreines 1.2-Dichlorethan flüssig abgezogen und ohne weitere Reinigung zur Herstellung von Vinylchlorid verwendet.

Es werden ähnlich gute Ausbeuten, wie in Beispiel 1 beschrieben, erzielt. Die Qualität des erzeugten 1.2-Dichlorethans entspricht der des Beispiels 1.

Vergleichsbeispiel 1

Unter sonst gleichen Bedingungen wie in Beispiel 1 werden 4 500 kg/Stunde Elektrolyse-Chlor über Leitung 2 mengengeregelt in den Reaktor 1 eingebracht. Zum Ausgleich der Wärmebilanz werden 12 t/Stunde Umlaufprodukt unter Umgehung des Chlorabsorbers 15 im Kreis gepumpt.

Das erhaltene Roh-1.2-Dichlorethan hat folgende Zusammensetzung :

2 700 Gew.ppm Ethylchlorid
900 Gew.ppm 1.1-Dichlorethan
2 Gew.% 1.1.2-Trichlorethan
4 500 Gew.ppm Tetra- und Pentachlorethane
Rest 1.2-Dichlorethan

Die oben beschriebene Verfahrensweise unterscheidet sich gegenüber dem erfindungsgemäßen Verfahren (Beispiel 1) im wesentlichen dadurch, daß das eingesetzte Elektrolyse-Chlor nicht in gelöster Form, sondern in seiner Hauptmenge in gasförmigen Zustand in den Reaktor gelangt. Dadurch erhöht sich vergleichsweise die Bildung von Nebenprodukten um den Faktor 10. Entsprechend ergibt sich eine Ausbeuteverminderung an Reinprodukt um ca. 2,8 %.

Der zu erzielende Produktaustrag beträgt 5 443 kg/Stunde Rein-1.2-Dichlorethan.

Vergleichsbeispiel 2

Es wird analog Beispiel 1 gearbeitet, jedoch ohne Zusatz von Kresolen zum umlaufenden Reaktionsmedium.

Folgende Rohproduktzusammensetzung wurde gemessen :

1 500 Gew.ppm Ethylchlorid
500 Gew.ppm 1.1-Dichlorethan
5 000 Gew.ppm 1.1.2-Trichlorethan
980 Gew.ppm Tetra- und Pentachlorethane
Rest 1.2-Dichlorethan

Entsprechend ergibt sich eine Ausbeuteverminderung an Rein-Dichlorethan um etwa 1,7 % gegenüber dem erfindungsgemäßen Verfahren (Beispiel 1). Es fallen nur noch 5 506 kg/Stunde an Reinprodukt an.

Beispiel 4

Die Wirkungsweise der erfindungsgemäß eingesetzten hydroxylgruppenhaltigen Aromaten vom Typ o-Kresol, m-Kresol, wird anhand eines Laborversuchs demonstriert :

In 1.1.2-Trichlorethan-freiem 1.2-Dichlorethan werden bei 20 °C und Atmosphärendruck 5 Gew.% Chlor gelöst, mit 10 Gew.ppm m-Kresol versetzt und unter Lichtausschluß mehrere Tage aufbewahrt. Die Probe wird anschließend auf ihren Gehalt an 1.1.2-Trichlorethan untersucht und mit einer gleichbehandelten Blindprobe (ohne Zusatz von Kresol) verglichen :

Die Proben werden zur Extraktion überschüssigen Chlors mit verdünnter Natronlauge geschüttelt, die organische Phase über wasserfreiem Natriumsulfat getrocknet und gaschromatographisch untersucht.

Ergebnisse

1.1.2-Trichlorethangehalt der Blindprobe : 300 ppm
1.1.2-Trichlorethangehalt der Probe (Mit Kresolzugabe) : unter 10 ppm.

**Ansprüche**

1. Verfahren zur Herstellung von 1.2-Dichlorethan durch Chlorierung von Ethylen in der Flüssigphase in Gegenwart von Lewissäurekatalysatoren, wobei die Hauptmenge der freiwerdenden Reaktionswärme zum Verdampfen und Rektifizieren des Reaktionsproduktes ausgenützt wird, dadurch gekennzeichnet, daß die Reaktion bei Drücken von 0,3 bis 1,3 bar und Temperaturen von 50 bis 90 °C durchgeführt wird, mindestens die Hälfte des benötigten Chlors gelöst im gekühlten Reaktionsprodukt zum Einsatz kommt und das restliche Chlor gasförmig und/oder flüssig zugegeben wird, das Reaktionsprodukt vor der Chlorabsorption mit 0,000 1 bis 0,01 Gew.% o-Kresol, m-Kresol und/oder deren Mono- bzw. Dichlorderivaten einzeln und/oder im Gemisch versetzt wird und die Wärmebilanz ausgeglichen wird durch die Maßnahmen :

a) Verdampfen des Reaktionsprodukts

b) Rektifizieren des Reaktionsprodukts bei 3- bis 5-fachem Rücklauf, bezogen auf den Produktaustrag vom Kondensator in den Kolonnenkopf

c) Ausschleusen des Reaktionsprodukts

d) Kühlen des Reaktionsprodukts in Mengen vom 1,5- bis zum 10-fachen des Produktaustrags und

e) Beschicken des gekühlten Reaktionsprodukts mit Chlor und Rückführen des chlorhaltigen, gekühlten Reaktionsprodukts in den Reaktionsraum.

2. Verfahren nach Anspruch 1, dadurch gekennzeichnet, daß die Reaktion bei einem Überschuß von 2 bis 10 % an Chlor, bezogen auf die Menge umzusetzenden Ethylens, durchgeführt wird.

3. Verfahren nach Anspruch 1, dadurch gekennzeichnet, daß ein Teilstrom des gekühlten, mit o-Kresol, m-Kresol und/oder deren Mono- bzw. Dichlorderivaten einzeln und/oder im Gemisch versetzten, Reaktionsprodukts zur Abgaswäsche verwendet und anschließend in die Reaktionszone zurückgeführt wird.

4. Verfahren nach Anspruch 1, dadurch gekennzeichnet, daß im Reaktor ein Umwälzkreislauf aufrechterhalten wird.

**Claims**

1. Process for the manufacture of 1,2-dichloroethane by chlorination of ethylene in the liquid phase in the presence of Lewis acid catalysts, wherein the main quantity of the liberated heat of reaction is exploited for the vaporization and rectification of the reaction product, characterized in that the reaction is carried out at pressures of from 0.3 to 1.3 bar and at temperatures of from 50 to 90 °C, at least half the quantity of chlorine required is used dissolved in the cooled reaction product and the remaining chlorine is introduced in the gaseous and/or liquid state, from 0.000 1 to 0.01 % by weight of o-cresol, m-cresol and/or the monochloro or dichloro derivatives thereof, alone and/or in admixture, is added to the reaction product prior to the chlorine absorption, and the thermal balance is maintained by the following measures :

   a) vaporization of the reaction product,

   b) rectification of the reaction product with an amount of product corresponding to from 3 to 5 times the amount discharged being returned from the condenser into the top of the column,

   c) discharging reaction product from the reactor,

   d) cooling an amount of the reaction product corresponding to from 1.5 to 10 times the amount discharged, and

   e) charging the cooled reaction product with chlorine and returning the chlorine-containing cooled reaction product to the reaction zone.

2. Process according to claim 1, characterized in that the reaction is carried out with an excess of from 2 to 10 % of chlorine, calculated on the amount of ethylene to be reacted.

3. Process according to claim 1, characterized in that part of the cooled reaction product, to which o-cresol, m-cresol and/or the monochloro or dichloro derivatives thereof, alone and/or in admixture, have been added, is used for washing the waste gas and is then returned to the reaction zone.

4. Process according to claim 1, characterized in that circulation is maintained in the reactor.

**Revendications**

1. Procédé de production de dichloro-1,2 éthane par chloration de l'éthylène en phase liquide en présence de catalyseurs qui sont des acides de Lewis, selon lequel la majeure partie de la chaleur libérée par la réaction sert à évaporer et à rectifier le produit de la réaction, procédé caractérisé en ce que :

on effectue la réaction sous des pressions de 0,3 à 1,3 bar et à des températures de 50° à 90 °C, on ajoute au moins la moitié du chlore nécessaire dissous dans du produit de réaction refroidi et l'on ajoute le reste du chlore sous forme gazeuse et/ou liquide,

on ajoute au produit de réaction, avant l'absorption du chlore, 0,000 1 à 0,01 % en poids d'o-crésol, de m-crésol et/ou de leurs dérivés mono- ou di-chlorés isolément ou en mélange, et on équilibre le bilan thermique par les mesures suivantes :

   a) évaporation du produit de la réaction,

   b) rectification du produit de la réaction avec un reflux représentant 3 à 5 fois le produit soutiré du condenseur en tête de colonne,

   c) éjection du produit de la réaction,

   d) refroidissement du produit de la réaction en des quantités représentant 1,5 à 10 fois la quantité de produit soutirée et

   e) fourniture de chlore au produit de réaction refroidi et recyclage vers la zone de réaction du produit de réaction refroidi et contenant du chlore.

2. Procédé selon la revendication 1, caractérisé en ce qu'on effectue la réaction avec un excès de 1 à 10 % en chlore, par rapport à la quantité d'éthylène à faire réagir.

3. Procédé selon la revendication 1, caractérisé en ce qu'on utilise pour laver les gaz d'échappement un courant partiel du produit de réaction refroidi et additionné de o-crésol, de m-crésol et/ou de leurs dérivés mono- ou di-chlorés, isolément ou en mélange, et en ce qu'on le renvoie ensuite dans la zone de réaction.

4. Procédé selon la revendication 1, caractérisé en ce qu'on maintient dans le réacteur un circuit de recyclage.